# EUROPEAN PATENT APPLICATION

(11) **EP 0 804 903 A2**
(43) Date of publication of application: **05.11.1997**
(21) Application number: 97202080.4
(22) Date of filing: 29.05.1992
(51) Int. Cl.: A61B 17/02

(54) **Endoscopic inflatable retraction devices and method of making**

(30) Priority: 29.05.1991 US 706781; 19.11.1991 US 794590
(62) Divisional of application: 92912904.7
(71) Applicant: ORIGIN MEDSYSTEMS, INC., Menlo Park, CA 94025 (US)
(72) Inventor: Moll, Frederic H., San Francisco, CA 94118 (US); Chin, Albert K., Palo Alto, CA 94303 (US); Gadacz, Thomas R., Augusta, CA 30909 (US)
(74) Representative: Schmitz, Jean-Marie

(57) **Abstract**

The present invention concerns an apparatus (2) for providing a retraction force from outside the body to retract first tissue (e.g. L) inside the body to gain access to adjacent tissue (e.g. GB), comprising an inflatable chamber means for engaging the first tissue and a shaft means (12) for manipulating the inflatable chamber means, the inflatable chamber means being provided by the inflatable portion of a balloon catheter having a flexible tube and a rigid stylet inserted into the flexible tube for providing together with the flexible tube, the shaft means (12).

## Description

The present invention concerns an apparatus for providing a retraction force from outside the body to retract first tissue inside the body to gain access to an adjacent tissue according to the precharacterizing portion of claim 1 and a method of making an inflatable retraction device according to the precharacterizing portion of claim 2.

The invention relates to devices for use in laparoscopic Surgery, in particular, to devices that provide retraction of an organ to gain access to treat or observe a tissue.

Laparoscopy dates back to the turn of the 20^{th} Century. Early laparoscopic techniques were used primarily for diagnostic purposes to view the internal organs, without the necessity of conventional surgery. Since the 1930s laparoscopy has been used for sterilization and, more recently, for the suturing of hernias. US-A-4,919,152 and 4,944,443 are concerned with techniques for suturing hernias. Another very recent innovation is the use of laparoscopic surgery for removing the gallbladder.

Published International Application No. WO-A-92/21294 under the Patent Cooperation Treaty, describes an apparatus and method wherein the abdominal wall is lifted away from the underlying abdominal organs by an inflatable device which is introduced laparoscopically and, once in place, inflated to engage and lift an extensive area of the abdominal wall.

Even when such lifting techniques are used, it is still necessary to retract other organs to gain access to the organ or tissue to be treated or observed. In other procedures, to gain access to the organ or tissue to be treated or observed, it is necessary to separate the organ to be treated from tissue surrounding it. For example, to be able to observe the outer surface of the heart, the outer surface of the heart has to be separated from the pericardium.

Published International Application No. WO-A 92/21293 under the Patent Cooperation Treaty, describes inflatable retraction devices that retract organs or tissues by means of an inflatable chamber. The retraction device is introduced in a collapsed state into the body through a small incision, and, once in place, inflated to engage an extensive area of the organ or tissue to be retracted, and to gently retract or displace the organ or tissue without damaging it. During laparoscopic treatment and observation procedures, the retraction device retains its expanded condition, and hence its ability to provide retraction, while providing access for surgical instruments through itself to the organ or tissue being treated or observed, or allowing an organ or tissue to be brought inside itself for observation or treatment.

The inflatable retraction devices described in WO-A 92/21293, and one of the inflatable retraction devices described in the present application, are placed in the body through a small incision, and, when inflated, retract the organ by pushing against other adjacent organs and tissues. The force exerted by the retraction device against other organs or tissues within the body cavity can sometimes cause trauma and even damage to the other organs. Hence, it is sometimes preferable to provide a retraction device that can introduced into the body through a small incision, and which provides retraction without exerting a force against other adjacent organs and tissues.

The publication DE-A-2 847 633 describes an apparatus for use inside a body comprising an inflatable chamber means for applying a force between a first organ and a second organ when in an inflated state, the inflatable chamber means being insertable in collapsed state, and an inflating means for inflating the inflatable chamber means when the latter is in place within the body.

To provide a retraction from outside the body, current laparoscopic procedures use several small metal or plastic retractors inserted though a plurality of incisions. The retractors are fixed to a suitable bar to hold them in place once the desired amount of retraction has been achieved. Because such retractors have a relatively small surface area (the retractors have to be small enough to fit through a small incision), they tend to damage and/or cause trauma to the retracted organs. Moreover, the required plurality of incisions in the body wall undoes some of the advantage of using laparoscopic techniques.

The apparatus for providing a retraction force from outside the body to retract a first tissue is defined in the characterizing portions of claim 1 and the method for making an inflatable retraction device is defined in the characterizing portion of claim 2.

The present invention relates to inflatable retraction devices that mechanically retract organs and tissues to provide access to treat or observe other organs or tissues. In the following description, the word "organ" will be used to mean an organ or a tissue that is retracted by the retraction device. The word "treat" will be used to mean both treat and observe, and the word "treatment" will be used to mean both treatment and observation. The word "tissue" or the phrase "tissue to be treated" will both be used to mean the organ or the tissue that is treated after the organ has been retracted.

The inflatable retraction device according to the invention provides a retraction force from outside the body to retract an organ inside the body to gain access to an adjacent tissue. The inflatable retraction device comprises an inflatable chamber that engages with the organ, and a shaft for manipulating the inflatable chamber to retract the organ. The shaft has a distal end to which the inflatable chamber is attached, a bore that communicates with the inflatable chamber and allows the inflatable chamber to be inflated to an expanded state when the inflatable chamber is in place within the body. The shaft has a proximal end, which remains outside the body when the inflatable chamber is in place in the body.

In a first variation, the inflatable chamber is substantially spherical and in a second variation, the inflatable retraction device comprises a balloon catheter with a stylet inserted into the tube of the balloon catheter.

In the second method for retracting, by means of a retraction force provided from outside the body, an organ in the body in the course of treating an adjacent tissue, an inflatable retractor is provided having an inflatable chamber, and a hollow shaft having a distal end and a proximal end. The inflatable chamber is attached to the distal end of the shaft in a collapsed state. A small incision is made in the body. The proximal end of the shaft is manipulated to pass the inflatable chamber and part of the shaft into the body through the small incision, and to place the inflatable chamber adjacent to the organ. A fluid is passed through the shaft to inflate the inflatable chamber into an expanded state. Finally, the proximal end of the shaft is manipulated to engage the organ with the inflatable chamber, and to retract the organ.

In a method according to the invention of making an inflatable retraction device, a balloon catheter having an inflatable chamber and a flexible tube is provided. A rigid stylet is also provided, and is inserted into the flexible tube of the balloon catheter.

In order that the invention may be fully understood, reference is made on the accompanying drawings, wherein
Figure 1A through 1E show an inflatable retraction device according to the invention that provides a retraction force from outside the body, wherein:
   Figure 1A shows the inflatable retraction device with its inflatable chamber in its collapsed state outside the shaft.
   Figure 1B shows the inflatable retraction device with its inflatable chamber in its collapsed state inside the shaft.
   Figure 1C shows the inflatable retraction device with its inflatable chamber in its expanded state.
   Figure 1D shows a version of the inflatable retraction device made using a modified Foley catheter in its collapsed state.
   Figure 1E shows a version of the inflatable retraction device made using a modified Foley catheter in its expanded state.
Figure 2A shows an inflatable retraction device according to the invention that provides a retraction force from outside the body with its inflatable chamber in its collapsed state, prior to insertion into the abdomen.
Figures 2B through 2E show a longitudinal cross-section of the abdomen with the inflatable retraction device shown in figure 2A, wherein:

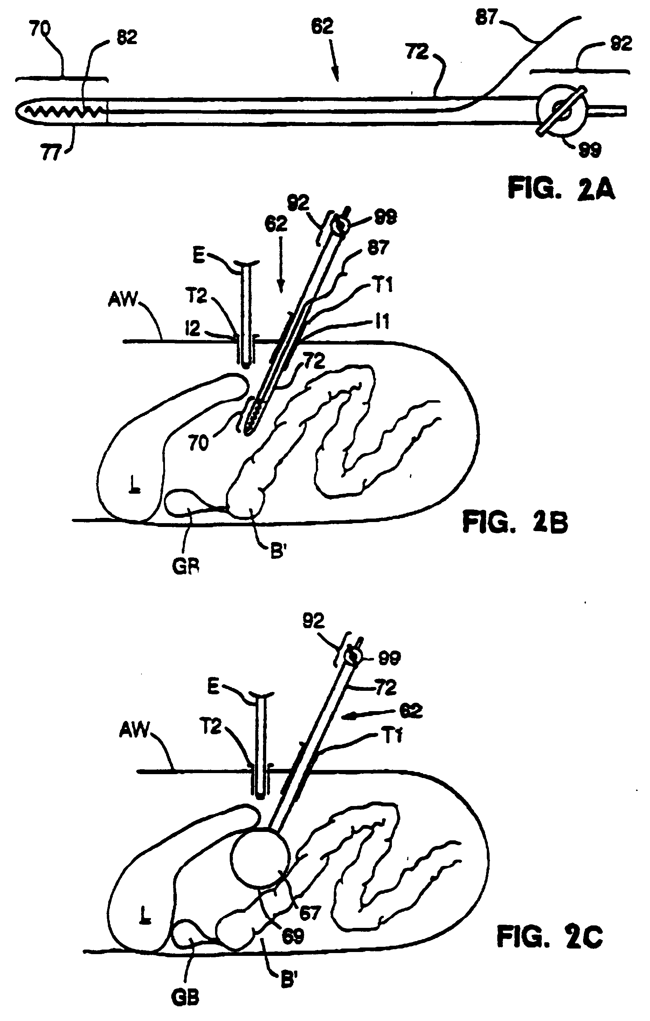

   Figure 2B shows the inflatable retraction device after insertion adjacent to the liver.
   Figure 2C shows the inflatable retraction device in contact with the liver after inflation of the inflatable chamber to its expanded state.

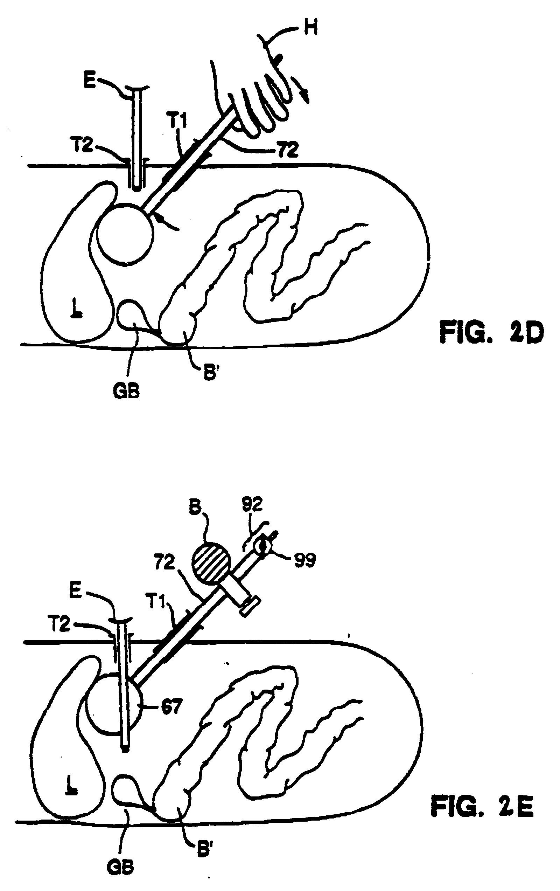

   Figure 2D shows the inflatable retraction device being manipulated to retract the liver.
   Figure 2E shows the inflatable retraction device clamped to a bar to maintain the liver in its retracted position.

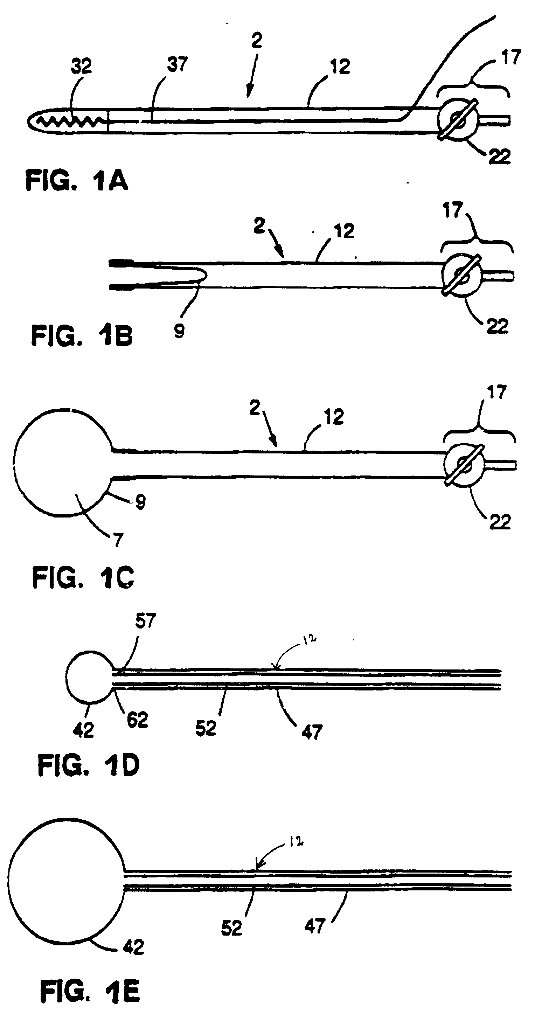

Figures 1A through 1E show some variations on an inflatable retraction device of the present invention that provides a retraction force from outside the body. The inflatable retraction device is inserted into a part of the body, such as the abdomen, through a trocar inserted into a single small incision about 10-20 mm long in the body wall. The inflatable retraction device provides a relatively large surface area over which a retraction force provided from outside the body is applied.

The inflatable retraction device 2, shown in its inflated state in figure 1C, comprises a small (about 50 mm (2") diameter) inflatable chamber 7 on the distal end 4 of a hollow cylindrical plastic or metal shaft 12 about 5 mm (0.2") in diameter. The inflatable chamber has an envelope 9 preferably of an elastomeric material, such as latex, but the envelope can be made of a non-elastic material such as polyethylene, polyurethane, Mylar R, or a polyethylene and nylon composite. The shaft 12 fits inside a standard 5.5 mm internal diameter trocar. The proximal end 14 of the shaft 12 includes a fitting 17 to which a source of inflation fluid (not shown) can be attached. The fitting 17 includes a valve 22 that enables the inflatable chamber 7 to be maintained in its inflated state when the source of inflation fluid is disconnected.

Figures 1A and 1B show the inflatable retraction device 2 with its inflatable chamber 7 in two alternative collapsed states. In figure 1A, the envelope (not shown) of the inflatable chamber is packed into a small volume and is held in its packed state by the sleeve 27. The sleeve 27 is held in place by the detachable lacing 32. The detachable lacing is detached by pulling the cord 37. Alternatively, the sleeve 27 can include a tear strip (not shown), or the envelope can be held in its packed state by detachable lacing alone, or by some other suitable means. The packed envelope forms a linear extension of the shaft 12.

In figure 1B, the envelope 9 of the inflatable chamber is attached to the distal end of the shaft 12 and a low vacuum is applied to the fitting 17 to draw the envelope up inside the bore 5 of the shaft 12. Once the envelope has been stored in the shaft 12, the vacuum may be released. Internal storage of the envelope of the inflatable chamber is preferred, and is particularly suitable if the envelope is made of an elastomeric material. With a non-elastomeric envelope, the envelope must be packed before it is drawn into the bore of the shaft. Internal storage cannot be used if the packed envelope is too bulky to fit in the bore of the shaft 12.

An inflatable retractor of the type just described for providing a retraction force from outside the body can be made by modifying a balloon catheter, such as a Foley catheter, as shown in figures 1D and 1E. A balloon catheter has an elastomeric inflatable chamber 42 on the end of a flexible catheter 47. The inflatable chamber of the balloon catheter provides the inflatable chamber 7 (figure 1C) of the inflatable retractor. The catheter of an unmodified balloon catheter, even when inflated, is too flexible to allow the balloon catheter to apply a useable retraction force. A modified balloon catheter according to the invention is shown in its collapsed state in figure 1D, and in its expanded state in figure 1E. The balloon catheter is modified by inserting a stylet 52 into the catheter 47. The stylet is preferably inserted such that the distal end 57 of the stylet substantially coincides with the distal end 62 of the catheter, i.e., the junction of the catheter 47 and the inflatable chamber 42. The rigid stylet together with the flexible catheter provides the shaft 12 (figure 1C) of the inflatable retraction device and enables the modified balloon catheter to be used to retract organs.

A method of using an inflatable retraction device of the type shown in figures 1A-1C to lift the liver so that the gall bladder can be observed will now be described with reference to figures 2A through 2E. The method can also be practiced using an inflatable retraction device made by modifying a balloon catheter, such as a Foley catheter.

The inflatable retraction device 62 is supplied with its inflatable chamber 67 in a collapsed state. The envelope (not shown) of the inflatable chamber is packed so that it forms a linear extension of the shaft 72, as shown in figure 2A. The envelope is retained in its packed state by the sleeve 77 and the detachable lacing 82. Alternatively, a sleeve with a tear strip, detachable lacing alone, or some other suitable means, can be used. alternatively, the envelope can be mounted inside the tube 72, as shown in figure 1B.

Referring to figure 2B, an incision I1 is made in the abdominal wall AW and a 5.5 mm external diameter trocar T1 is driven through the abdominal wall. A second incision I2 is made so that a suitable endoscope E can be inserted into the abdomen through an additional trocar T2. The cord 87 attached to the detachable lacing 82 is run along the length of the tube T2.

The proximal end of the shaft 72 is then grasped and manipulated to insert the packed envelope 70 and the distal part of the shaft into the abdomen through the trocar T1. Once the packed envelope has passed through the trocar T1, the proximal end of the shaft 72 is manipulated to bring the packed envelope 70 close to the liver L. The shaft is then temporarily clamped in position by attaching it to a suitable bar (not shown).

The cord 87 is pulled to detach the detachable lacing 82 from the sleeve 77. This releases the sleeve from around the packed envelope 70. The cord and detachable lacing remain attached to the sleeve so that the sleeve can be withdrawn from the abdominal cavity through the trocar T1, either immediately or at the of the operation.

A source of inflation fluid is attached to the fitting 92. The preferred inflation fluid is air, although a different gas, such as carbon dioxide, or a liquid, such as saline solution, can be used. The valve 99 is turned on to enable inflation fluid to flow through the bore of the shaft 72 into the inflatable chamber 67. This releases the envelope 69 from its packed state and inflates the inflatable chamber into its expanded condition, as shown in figure 2C. When the inflatable chamber is fully expanded, the valve 99 is preferably turned off and the source of inflation fluid is disconnected. Alternatively, the source of inflation fluid can be left connected and the valve 99 left turned on.

The proximal end of the shaft 72 is gripped by the hand H, the shaft 72 is detached from the bar, and, while observing through the endoscope E, the proximal end of the shaft 72 is manipulated to engage the inflated inflatable chamber 67 with the liver L. The shaft 72 is then further manipulated to push the inflated inflatable chamber 67 against the liver as shown in figure 2D. The force applied to the liver by the relatively large area of the inflatable chamber gently retracts the liver so that the gall-bladder GB can be seen through the endoscope E. When the liver is suitably retracted, the shaft 72 is once more clamped to the bar B to hold the liver in its retracted condition, as shown in figure 2E.

After observation has been completed, the proximal end of the shaft 72 is once more gripped and the shaft is released from the bar B. The shaft is then manipulated to allow the liver to return to its normal, non-retracted position. The valve 99 is operated to release the inflation fluid from the inflatable chamber. The fitting 92 is also preferably connected to a low vacuum to further collapse the inflatable chamber 67. With the inflatable chamber fully collapsed, the valve 99 is returned to its off position. The proximal end of the shaft 72 is manipulated to withdraw the inflatable chamber 67 and shaft 72 from the abdomen through the trocar T1. The cord 87, detachable lacing 82, and sheath 77 are withdrawn form the abdomen through the trocar T1 by pulling on the cord 87. Finally, the trocars are withdrawn from their respective incisions.

## Claims

1. Apparatus (2) for providing a retraction force from outside the body to retract first tissue (e.g. L) inside the body to gain access to adjacent tissue (e.g. GB), said apparatus comprising inflatable chamber means for engaging the first tissue and shaft means (12) for manipulating the chamber means including a distal end (57) attached to the chamber means, bore means communicating with the chamber means to inflate the chamber means to an expanded state when in place with the body and a proximal end for disposition outside the body when the chamber means is in place within the body; characterized in that the chamber means is provided by the inflatable portion (42) of a balloon catheter (42 & 47) having a flexible tube (47) and that a rigid stylet (52) inserted into the flexible tube provides, together with the flexible tube, the shaft means (12).

2. A method of making an inflatable retraction device for providing a retraction force from outside the body to retract first tissue inside the body to gain access to adjacent tissue; characterized in providing a balloon catheter (42 & 47) having an inflatable chamber (42) and a flexible tube (47), providing a rigid stylet (52), and inserting the rigid stylet into the flexible tube.

3. The method of claim 2 characterized in that
the flexible tube (47) has a distal end (57) adjacent to the inflatable chamber (42),
the rigid stylet (52) has a distal end (62) and
in the step of inserting the stylet (52) the flexible tube, the stylet (52) is inserted such that the distal end of the stylet substantially coincides with the distal end (57) of the flexible tube.
